# EUROPÄISCHE PATENTANMELDUNG

(11) **EP 0 742 026 A1**
(43) Veröffentlichungstag der Anmeldung: **13.11.1996**
(21) Anmeldenummer: 96107455.6
(22) Anmeldetag: 10.05.1996
(51) Int. Cl.: A61M 16/00, A61B 1/00

(54) **Endoskop, insbesondere für die Intubation**

(30) Priorität: 12.05.1995 DE 29507905 U
(71) Anmelder: Werner, Wolfgang, Dr. med., 34497 Korbach (DE)
(72) Erfinder: Werner, Wolfgang, Dr. med., 34497 Korbach (DE)
(74) Vertreter: Freiherr von Schorlemer, Reinfried, Dipl.-Phys.

(57) **Zusammenfassung**

Die Erfindung betrifft ein Endoskop mit einem Halteteil (1), mit dem ein flexibles Teil (2) verbunden ist, in dem wenigstens je ein der Beleuchtung dienendes und ein der Betrachtung dienendes, lichtleitendes Faserbündel (3,4,5) angeordnet sind. Das eine Faserbündel (3) ist mit einem am Halteteil (1) montierten Anschluß (6) für eine Lichtquelle, das andere Faserbündel (5) mit einer am Halteteil (1) montierten Betrachtungsoptik (8) verbunden. Das Teil (2) ist erfindungsgemäß wenigstens teilweise formbar ausgebildet.

## Beschreibung

Die Erfindung betrifft ein Endoskop der im Oberbegriff des Anspruchs 1 angegebenen Gattung.

Endoskope sind Instrumente zum Besichtigen enger Körperhöhlen. Sie bestehen in der Regel aus einem in die Körperhöhle einzuführenden, starren oder flexiblen Rohr oder Schlauch, in dem zur Beleuchtung und Betrachtung bestimmte Glasfaserbündel angeordnet sind (DE 24 54 370 B2, DE 29 50 520 C2). In Abhängigkeit von der zu untersuchenden Körperhöhle gibt es zahlreiche unterschiedliche Endoskoptypen. Die Erfindung befaßt sich insbesondere mit Endoskopen für die sogenannte fiberoptische Intubation, obwohl die der Erfindung zugrunde liegenden Probleme auch bei anderen Endoskopiearten auftreten können.

Unter Intubation wird das Einführen eines aus Gummi oder Kunststoff bestehenden Schlauchs (Tubus) in den Kehlkopf und weiter in die Trachea des Individiums verstanden. Sie dient der Aufrechterhaltung eines effektiven, u.a. für die Sauerstoffversorgung der Organe lebensnotwendigen Gasaustauschs, der normalerweise durch Atmung erfolgt. In Situationen, in denen der Patient infolge Krankheit, Verletzung oder Medikamentengabe, z.B. bei der Durchführung einer Narkose, nicht mehr in der Lage ist, die erforderliche Atemarbeit zu bewältigen, muß eine Beatmung durchgeführt werden. Voraussetzung für diese Beatmung ist eine sichere Verbindung zwischen Beatmungsgerät und den Atemwegen des Patienten. Um eine ausschließliche Belüftung der Atmungsorgane zu erreichen, ohne daß Luft über die Speiseröhre in den Verdauungstrakt gelangt, wird der Tubus mit seinem distalen Ende über den Mund oder die Nase in die Luftröhre geschoben und dort so plaziert, daß beide Lungenflügel belüftet werden. Am proximalen Ende wird der Tubus über ein genormtes Ansatzstück (Konnektor) mit dem Schlauchsystem des Beatmungsgeräts verbunden.

Liegt die Tubusspitze nicht in der Trachea, werden die Lungenflügel nicht belüftet. Das durch die Lunge strömende Blut wird dann nicht ausreichend mit Sauerstoff angereichert, und die Versorgung der Organe mit Sauerstoff bricht zusammen. Je nach der Dauer der ungenügenden Versorgung mit Sauerstoff sind neben einer vollständigen Wiederherstellung sämtlicher Körperfunktionen bleibende Hirnschäden (z.B. Koma) oder der Tod infolge Herzstillstand möglich. Dieselben Folgen ergeben sich bei Fehlintubationen, worunter Intubationsmanöver verstanden werden, bei denen der Tubus nicht richtig plaziert wird und z.B. die Tubusspitze in der Speiseröhre anstatt in der Luftröhre liegt.

Zur sicheren Plazierung der Tubusspitze gibt es mehrere Methoden und Tubusformen. Bei Patienten ohne anatomische oder pathologische Besonderheiten ist die Intubation in der Regel mit den gebräuchlichen Methoden, hauptsächlich der laryngoskopischen Intubation, einfach und schnell durchführbar. Schwierigkeiten ergeben sich allerdings z.B. beim Vorliegen von pathologischen Veränderungen oder anatomischen Besonderheiten, wodurch dann am nicht mehr atmenden Patienten die Kombination der laryngoskopischen Intubation mit der fiberoptischen Intubation oder die Anwendung von diesen speziell zugeordneten Geräten erforderlich wird.

Bei der laryngoskopischen Intubation werden der Zungengrund und Kehlkopfdeckel des Patienten mit einem Laryngoskop angehoben, um eine freie Sicht auf den Kehlkopfeingang, die Stimmritze, zu erhalten. Ist dabei die Stimmritze nur teilweise einsehbar, dann ist die Einführung des Tubus schwierig. Man behilft sich damit, daß die Form des Tubus in seiner Längsachse so lange verändert wird, bis die Tubusspitze den Tracheaeingang sicher erreichen kann. Dies geschieht bisher durch eine innere Schienung des Tubus mittels eines in diesen eingesetzten Führungsstabs in Form eines mit Kunststoff ummantelten biegsamen Drahts, der nach dem Biegevorgang so stabil ist, daß er seine Form in der Längsachse auf den elastischen Tubus überträgt. Er ist im Tubus in der Längsachse verschiebbar, so daß er auch mit seiner weichen Spitze aus dem distalen Tubusende herausragen kann. Je nach den anatomischen Gegebenheiten wird der Führungsstab so gebogen, daß seine Spitze durch die Stimmritze geschoben und anschließend die Tubusspitze durch Schieben über den Führungsstab in der Trachea plaziert werden kann. Ist aufgrund anatomischer Schwierigkeiten die Stimmritze nicht einsehbar, so daß der Weg des Tubus bzw. Führungsstabs beim Vorschieben nicht beobachtet werden kann, liegt bei erhöhtem Verletzungsrisiko eine stark erniedrigte Trefferquote vor. Für diese Fälle gibt es zwar Spezialinstrumente, die auch unter schwer einstellbaren Bedingungen einen Blick auf die Stimmritze ermöglichen sollen, doch wird bei deren Anwendung wegen der meistens beengten Verhältnisse die Sicht durch den Tubus häufig behindert. Die Anwendung der laryngoskopischen Intubation ist daher in schwierigen Fällen nicht optimal.

In derartigen Fällen wird daher vorzugsweise die fiberoptische Intubation angewendet, bei der man sich zum Auffinden des Tracheaeingangs eines Endoskops der eingangs bezeichneten Gattung bedient, um das Beobachtungsfeld auszuleuchten und einzusehen. Außerdem kann mittels einer am Halteteil des Endoskops montierten Mechanik die Lage der Endoskopspitze verändert und dadurch unter Sicht durch die Stimmritze geschoben werden. Der vorher auf den Endoskopschlauch aufgeschobene Tubus wird dann bis in die Trachea vorgeschoben, worauf das Endoskop aus dem richtig plazierten Tubus herausgezogen und letzerer am Kopf des Patienten fixiert und an das Beatmungsgerät angeschlossen wird.

Auch die fiberoptische Methode ist nicht in allen Fällen optimal durchführbar. Sie ist insbesondere beim liegenden Patienten mit erschlaffter Muskulatur sehr schwierig, weil der Zungengrund nach hinten fällt und dadurch der Weg zur Trachea verlegt ist. Da außerdem bei der fiberoptischen Intubation eine Hand zum Führen des Endoskops, meistens mittels des umgebenden Tubus, und eine zweite Hand zum Bedienen der Endoskopmechanik benötigt wird, ist für das Anheben des Zugengrunds mittels eines Laryngoskops eine Hilfsperson erforderlich.

Der Erfindung liegt die Aufgabe zugrunde, das Endoskop der eingangs bezeichneten Gattung so auszubilden, daß es die Vorteile beider genannten Methoden weitgehend in sich vereinigt, ohne auch deren Nachteile zu übernehmen.

Zur Lösung dieser Aufgabe dienen die kennzeichnenden Merkmale des Anspruchs 1.

Die Erfindung bringt den Vorteil mit sich, daß der Anästhesist mit einer Hand z.B. den Zungengrund mit dem Laryngoskop anheben und mit der anderen Hand den Tubus unter direkter Sichtkontrolle plazieren und dann in die Trachea vorschieben kann. Da er weder zur Aufrechterhaltung der Form noch zur Betätigung der Mechanik eine Hand benötigt, ist er nicht mehr auf eine Hilfsperson angewiesen. Die Erfindung eignet sich daher vor allem in Fällen, in denen die Kombination beider Verfahren erforderlich ist.

Weitere vorteilhafte Merkmale der Erfindung ergeben sich aus den Unteransprüchen.

Die Erfindung wird nachfolgend in Verbindung mit den beiliegenden Zeichnungen an Ausführungsbeispielen näher erläutert. Es zeigen:
Fig. 1 eine perspektivische Darstellung eines erfindungsgemäßen Endoskops;
Fig. 2 einen Schnitt längs der Linie II-II der Fig. 1;
Fig. 3 einen Längsschnitt durch das distale Ende des Endoskops nach Fig. 1 und 2;
Fig. 4 und 5 ausgewählte, formstabile Biegezustände eines flexiblen Teils des erfindungsgemäßen Endoskops;
Fig. 6 einen Schnitt längs der Linie II-II der Fig. 1, jedoch durch eine zweite Ausführungsform der Erfindung;
Fig. 7 und 8 Schnitte entsprechend Fig. 2 durch verformbare Teile von zwei weiteren Ausführungsformen der Erfindung;
Fig. 9 und 10 in je einer schematischen, teilweise geschnittenen Ansicht sowie einem Längsschnitt durch eine Einzelheit eine weitere Ausführungsform der Erfindung; und
Fig. 11 und 12 in je einem schematischen Längsschnitt und Querschnitt noch eine andere Ausführungsform der Erfindung.

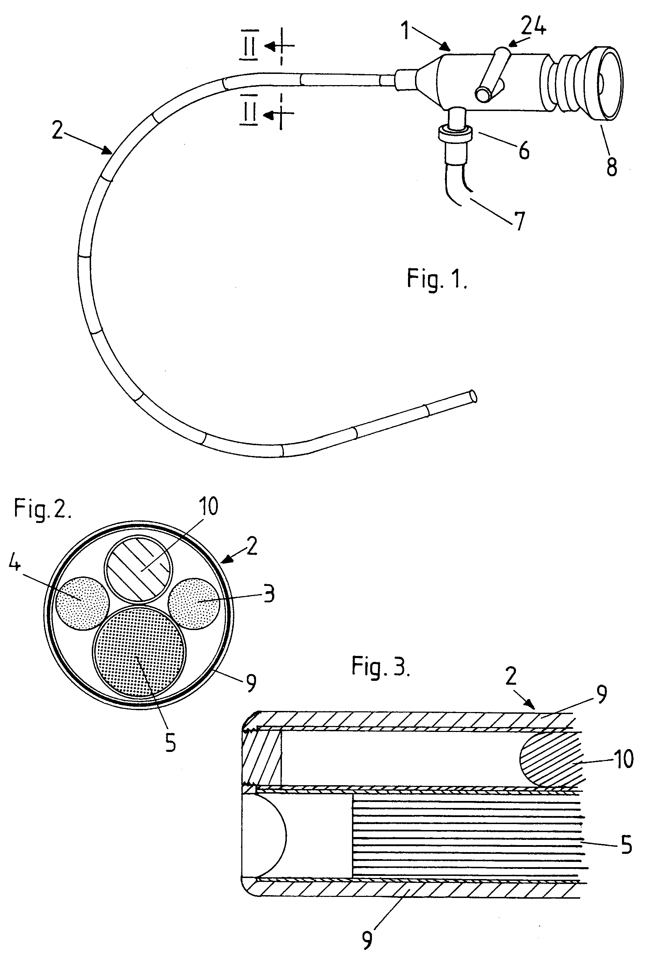

Das Endoskop enthält nach Fig. 1 bis 3 ein festes Halteteil 1 und ein flexibles, röhrenförmiges, als Einführungsschlauch ausgebildetes Teil 2 von länglicher Form, das mit einem Ende am Halteteil 1 befestigt ist. Im Teil 2 sind, wie Fig. 2 beispielsweise zeigt, zwei lichtleitende, der Beleuchtung dienende Faserbündel 3 und 4 und ein weiteres lichtleitendes, der Betrachtung dienendes Faserbündel 5 angeordnet. Dabei sind die Faserbündel 3 und 4 innerhalb des Halteteils 1 mit einem an diesem montierten Anschluß 6 verbunden, der z.B. über ein Lichtleiterkabel 7 an eine nicht gezeigte Lichtquelle angeschlossen wird, während ihr distales Ende im wesentlichen am distalen Ende des Teils 2 liegt und bei Bedarf mit wenigstens je einer Frontlinse verbunden sein kann. Das Faserbündel 5 ist innerhalb des Halteteils 1 mit einer an diesem montierten Betrachtungsoptik 8, z.B. einem nicht dargestellten, einstellbaren Okular, verbunden und mit seinem distalen Ende ebenfalls im Bereich des distalen Endes des Teils 2 angeordnet. Dabei kann das distale Ende des Faserbündels 5 bei Bedarf ebenfalls mit einer Frontlinse, z.B. in Form eines Objektivs od. dgl., versehen sein, die mit den Frontlinsen der Faserbündel 3 und 4 zu einer Frontlinseneinheit zusammengefaßt sein kann.

Im übrigen ist das beschriebene Endoskop in bekannter Weise ausgeführt und braucht daher dem Fachmann nicht näher erläutert werden. Dabei versteht sich, daß die Faserbündel 3, 4 und 5 jeweils mit nicht näher dargestellten, bei Lichtleitern üblichen Ummantelungen versehen sein können und statt eines der Faserbündel 3 bzw. 4, z.B. anstelle des Faserbündels 4, auch ein Instrumentenkanal oder ein Kanal zum Durchströmen eines Gases ausgebildet sein kann, das auf die Frontlinse umgelenkt wird, um deren Beschlagen zu verhindern.

Erfindungsgemäß ist das Teil 2 ein formbares Teil. Darunter wird für die Zwecke der Erfindung verstanden, daß es längs seiner Längsachse aus der üblichen, z.B. geraden Form in eine gebogene Form gebracht werden kann, indem es beispielsweise quer zur Längsachse gebogen wird, ohne daß es die Neigung besitzt, nach dem Loslassen wieder in die gerade oder irgendeine andere Vorzugsstellung zurückzukehren. Die im Einzelfall hergestellte Form soll vielmehr weitgehend stabil sein und nur durch entsprechende Manipulationen am Teil 2 wieder geändert werden könnnen.

Zu diesem Zweck sind nach einer Ausführungsform der Erfindung zunächst die Faserbündel 3 bis 5 und die sie ggf. einhüllenden Ummantelungen derart ausgebildet und angeordnet, daß sie bei einer Verformung um quer zu ihrer Längsachse gerichtete Achsen eine nur geringfügige Rückstellkraft besitzen. Entsprechend ist eine äußere Umhüllung 9 ausgebildet, von der die Faserbündel 3 bis 5 insbesondere zum Schutz umhüllt sind (Fig. 2). Die Umhüllung 9 ist mehrschichtig dargestellt und besteht z.B. aus einer mit Gummi beschichteten Metallfaserröhre, in der die Fasern, ähnlich wie bei einem Druckschlauch, spiralförmig angeordnet oder verflochten sind. Außerdem ist erfindungsgemäß in der Umhüllung 9 zusätzlich ein Formstab 10 angeordnet, der vorzugsweise über die ganze Länge des Teils 2 erstreckt und mit einem Ende am Halteteil 1 befestigt ist, während sein anderes Ende nahe am distalen Ende des Teils 2 liegt. Alternativ wäre es allerdings auch möglich, den Formstab 10 zumindest begrenzt verschiebbar in der Umhüllung 9 anzuordnen. Der Formstab 10 ist flexibel formbar, gleichzeitig aber auch formstabil, damit er manuell in eine Vielzahl von unterschiedlichen Biegestellungen gebracht werden kann und diese Stellungen nach dem Loslassen auch beibehält. Der Formstab 10 kann daher z.B. ein biegbarer Draht sein, der aus einem solchen Material besteht, daß durch Biegung dieses Drahtes praktisch jede im Rahmen einer Intubation erforderliche Biegestellung des Teils 2 hergestellt werden kann, und ist zweckmäßig mit einer faserverstärkten Ummantelung od. dgl. versehen, um Druckkräfte auf die Lichtleiter zu vermeiden. Geeignet sind vor allem Materialen, insbesondere Metalle, wie sie bisher zur inneren Schienung des Tubus verwendet werden. Der Querschnitt des Formstabs 10 muß dabei so groß sein, daß etwaige Rückstellkräfte, die aus entsprechenden Verbiegungen der Faserbündel 3 bis 5, der Umhüllung 9 und des sie umgebenden Tubus resultieren, keine Änderung einer gewünschten und hergestellten Biegestellung des Formstabs 10 herbeiführen können, so daß der jeweilige Biegezustand des Formstabs 10 gleichbedeutend mit einem entsprechenden Biegezustand des gesamten Teils 2 ist. Am distalen Ende sollte der Formstab 10 zur Herabsetzung des Verletzungsrisikos nicht sehr starr sein und ggf. geringfügig von der Umhüllung 9 überragt werden (Fig. 3).

Vor der eigentlichen Anwendung des beschriebenen Endoskops zur Intubation wird zunächst in an sich bekannter Weise ein ausreichend flexibler Trachealtubus auf das Teil 2 aufgezogen (z.B. DE 29 50 520 C2), ggf. nach Benetzung der Innenwand des Tubus und/oder der Außenwand des Teils 2 mit einem geeigneten Gleitmittel. Bei Bedarf wird der Tubus dann über einen geeigneten Konnektor an ein nicht gezeigtes Beatmungsgerät angeschlossen, damit er von einem Gasgemisch mit hohem Sauerstoffanteil durchströmt werden kann. Die Länge des formbaren Teils 2 wird zweckmäßig so gewählt, daß sein distales Ende bzw. die Frontlinseneinheit im voll aufgeschobenen Zustand des Trachealtubus über die Tubusspitze nach außen vorsteht. Dabei versteht sich, daß sowohl das Teil 2 als auch der Trachealtubus in unterschiedlichen Längen vorgesehen werden können. Außerdem werden die Frontlinsen zweckmäßig mit einem Antibeschlagmittel benetzt, worauf der Anschluß 6 an die Lichtquelle angeschlossen und diese eingeschaltet wird.

Das formbare Teil 2 wird nun in Abhängigkeit vom Patienten in eine solche Form vorgeformt, wie es für eine schnelle, verletzungsfreie Einführung der Tubusspitze in die Trachea sinnvoll erscheint. Sollte daraufhin die Intubation nicht sofort gelingen, wird die Form des Teils 2 so oft korrigiert, u.a. auch durch Abstützen an festen Strukturen im Rachenraum, bis die Intubation sicher durchgeführt werden kann. Mit Hilfe der Betrachtungsoptik 8 kann dabei jederzeit kontrolliert werden, daß die Tubusspitze richtig plaziert wird. Anschließend wird das formbare Teil sofort aus dem Tubus herausgezogen und der Tubus in üblicher Weise fixiert. Dies ist aufgrund der Verformbarkeit des formbaren Teils 2 bei Anwendung geringer Zugkräfte ohne weiteres möglich.

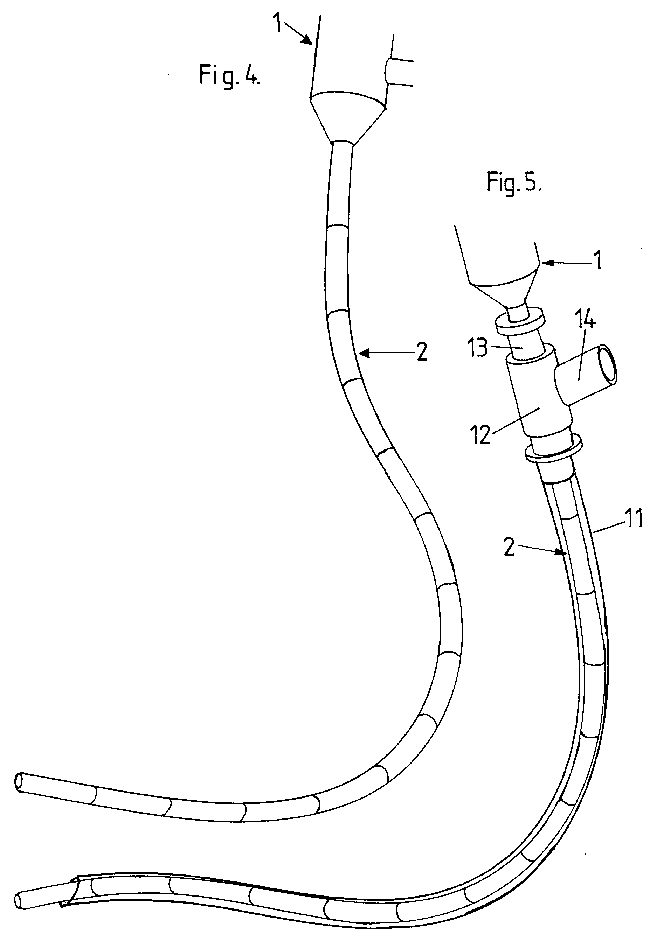

Fig. 4 und 5 zeigen beispielsweise zwei Biegestellungen des formbaren Teils 2, die sich in Abhängigkeit von den pathologischen oder anatomischen Gegebenheiten beim Patienten für die Intubation als sinnvoll erweisen können. Dabei bietet die erfindungsgemäße Kombination aus Endoskop und formbarem Teil 2 den Vorteil, daß unabhängig vom jeweiligen Biegezustand des Teils 2 stets eine Betrachtung des beleuchteten Mund- bzw. Rachenraums und damit auch in ganz schwierigen Fällen eine schnelle, gefahrlose und sichere Intubation möglich ist. Dabei versteht sich, daß die Stärke des Formstabs 10 und seine Formbeständigkeit so gewählt sind, daß auch bei aufgeschobenem Tubus der gewünschte Biegezustand hergestellt und erhalten werden kann. Alternativ wäre es natürlich auch möglich, mehr als einen Formstab 10 innerhalb der Umhüllung 9 anzuordnen. In Fig. 5 ist außerdem ein auf das Teil 2 aufgeschobener Tubus 11 mit einem Konnektoransatzstück 12 dargestellt, das eine Endoskopkappe 13 und einen Anschluß 14 für einen Beatmungsschlauch aufweist.

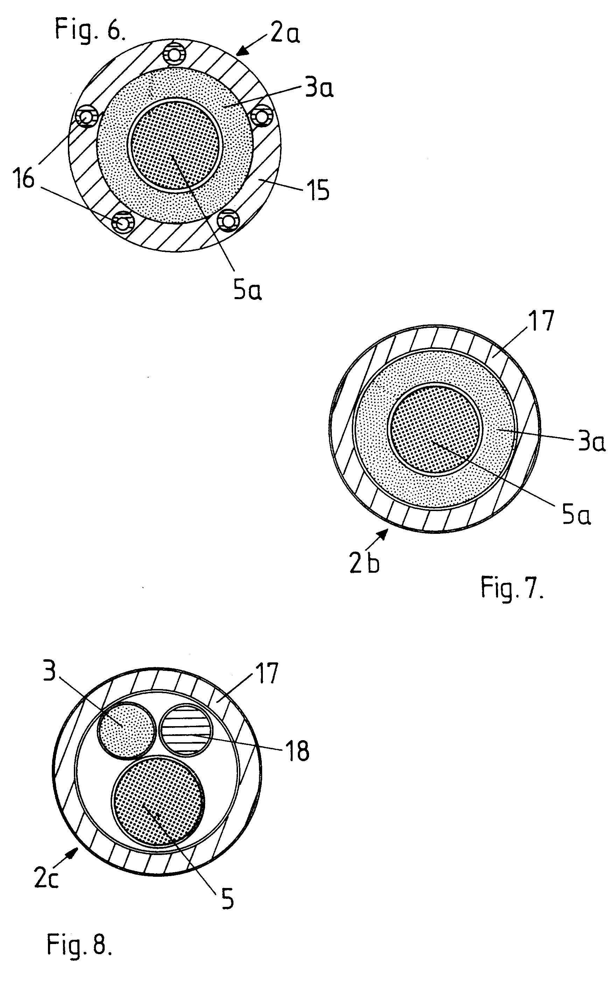

Bei einer zweiten, in Fig. 6 angedeuteten Ausführungsform der Erfindung ist ein formbares Teil 2a mit einer rohrförmigen, vergleichsweise dickwandigen Umhüllung 15 versehen, in der abweichend von Fig. 2 zwei konzentrisch angeordnete Faserbündel 3a und 5a vorgesehen sind, von denen das eine (3a) der Beleuchtung und das andere (5a) der Betrachtung dient. Abweichend von Fig. 2 ist eine Mehrzahl von formbaren Formstäben 16 direkt in das Material der Umhüllung 15 eingearbeitet und in deren Längsrichtung erstreckt. Im Ausführungsbeispiel sind fünf solche Formstäbe 16 vorhanden, doch können auch mehr oder weniger Formstäbe 16 vorgesehen sein, sofern alle Formstäbe 16 zusammen die gewünschte Formbarkeit und Formstabilität des Teils 2a ermöglichen.

Weiter liegt es im Rahmen der Erfindung, die Umhüllung 9 bzw. 15 selbst oder etwa vorhandene Ummantelungen der Faserbündel 3, 4 und 5 aus einem formbaren Material herzustellen, sofern dadurch die erforderliche relative Lage der Faserbündel 3, 4 und 5 zueinander nicht beeinträchtigt wird oder auf andere Weise sichergestellt werden kann.

Fig. 7 zeigt einen Querschnitt analog zu Fig. 2 und 6 durch eine Ausführungsform, bei der die Formstabilität eines Teils 2b allein aus der Wandbeschaffenheit einer Umhüllung 17 resultiert, die hier z.B. aus einem sog. Schwanenhals bzw. Lichthalterschlauch, d.h. einem spiralförmig gewundenen Metallband besteht, dessen Windungen sich jeweils berühren. Die Faserbündel 3a, 5a entsprechen denen nach Fig. 6. Dagegen zeigt Fig. 8 in einem entsprechenden Schnitt eine Ausführungsform eines Teils 2c mit der Ummantelung 17 nach Fig. 7, mit Faserbündeln 3 und 5 entsprechend Fig. 2 und mit einem Faserbündel 18, das entsprechend dem Faserbündel 4 auch als Instrumenten- oder Strömungskanal ausgebildet sein kann.

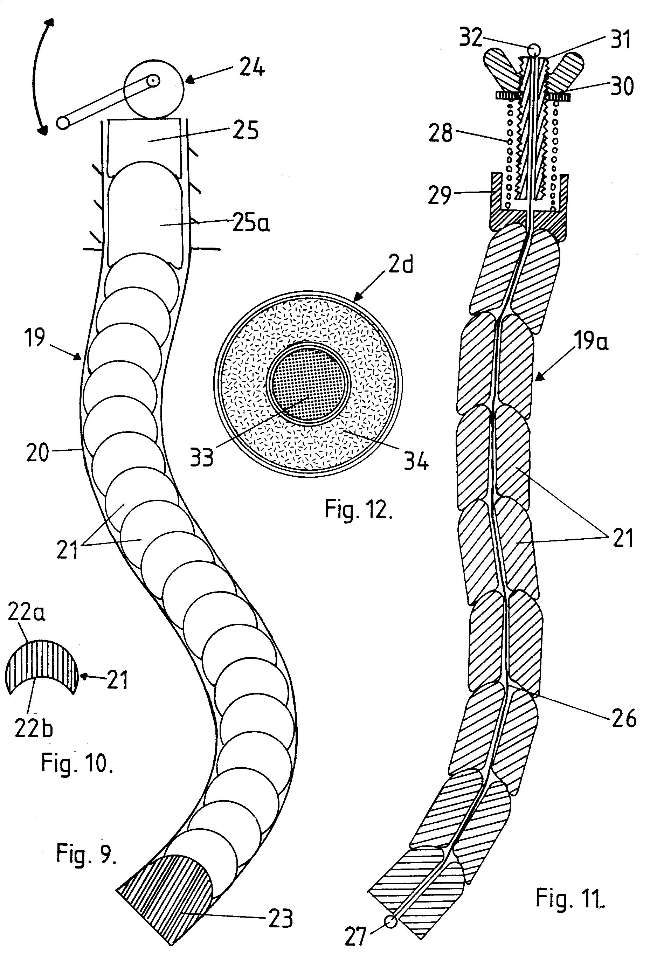

Bei einer weiteren, aus Fig. 9 und 10 ersichtlichen Ausführungsform ist anstelle der Formstäbe 10, 16 ein Formstab 19 vorgesehen, der eine am proximalen Ende verankerte Ummantelung 20 aus einem nicht dehnbaren, aber biegeschlaffen bzw. biegbaren Material aufweist. Die Formstabilität wird hier mittels einer Vielzahl von Elementen 21 erhalten, die in Längsrichtung des Formstabs 19 hintereinander in die Ummantelung 20 eingesetzt sind und an einer Grenzfläche 22a jeweils konvex, an der anderen Grenzfläche 22b dagegen konkav ausgebildet sind, wie insbesondere der Schnitt nach Fig. 10 durch ein Element 21 zeigt. Aneinandergrenzende konvexe und konkave Grenzflächen 22a, 22b grenzen dabei jeweils paarweise aneinander und bilden dadurch eine Art von Gliederstab, der durch die Ummantelung 20 zusammengehalten ist und in viele verschiedene Formen gebogen werden kann. Das distale Ende dieses Stabes ist fest mit der Ummantelung 20 verbunden, bespielsweise mittels eines entsprechend geformten Endstücks 23. Am proximalen Ende ist dagegen eine nur grob schematisch angedeutete Mechanik 24 (vgl. auch Fig. 1) vorgesehen, die auf ein oberstes Element 21 einwirkt und dazu dient, den Anpreßdruck in den Grenzflächen zwischen den einzelnen Elementen 21 festzulegen bzw. veränderbar zu machen. Dadurch kann eine durch Einwirkung von außen auf die Ummantelung 20 hergestellte Biegestellung des Formstabs 19 mittels der Mechanik 24 arretiert werden. Die hier als Exzentermechanik dargestellte Mechanik 24 kann in an sich beliebiger Weise ausgebildet sein und würde in der Gesamtdarstellung nach Fig. 1 an der dort bezeichneten Stelle des Halteteils 1 zu liegen kommen. Die Kraftübertragung könnte außerdem mittels eines zwischen das letzte Element 21 und die Mechanik 24 gelegten Zwischenstückes 25 und ggf. mittels eines zusätzlichen, zwischen das letzte Element 21 und das Zwischenstück 25 gelegten Zwischenstücks 25a aus einem vorzugsweise nicht völlig starren Material erfolgen.

Einen den Fig. 9 und 10 ähnlichen Formstab 19a zeigen die Fig. 11 und 12 bei weggelassener Ummantelung 20 in Fig. 11. Hier sind die einzelnen Elemente 21 zusätzlich mit wenigstens je einer Bohrung, insbesondere einer Mittelbohrung versehen und auf einen gemeinsamen Zugfaden 26 aufgezogen, dessen aus dem distalen Ende herausragendes Ende mit einem Knoten 27 od. dgl. gesichert ist. Am proximalen Ende wird der Druck mittels einer als Druckfeder ausgebildeten Schraubenfeder 28 auf ein zwischen ihr und dem letzten Element 21 angeordnetes Zwischenstuck 29 übertragen, wobei die Schraubenfeder 28 mit einem Ende auf dem Zwischenstück 29 und mit dem anderen Ende an einer Flügelmutter 30 abgestützt ist. Diese ist auf eine vom Zugfaden 26 durchragte Hülse 31 mit Außengewinde aufgedreht, wobei das aus der Hülse 31 herausragende Ende des Zugfadens 26 wiederum mit einem Knoten 32 od dg. gesichert ist. Je nach Lage der Flügelmutter 30 übt die Schraubenfeder 28 einen größeren oder kleineren Druck auf das Zwischenstück 29 bzw. die einzelnen Elemente 21 aus, so daß wahlweise eine beliebige Form des Gliederstabs hergestellt oder diese Form gesichert werden kann. Bei transparenter Ausbildung der Elemente 21 und Ausbildung des Zugfadens 26 als ummanteltes Lichtfaserbündel wäre es sogar möglich, beide zur Beleuchtung bzw. Beobachtung anzuwenden. Dies ist in Fig. 12 durch einen Faserbündel 33 und einen diesen konzentrisch umgebenden Gliederstab 34 angedeutet. Weiter wäre es möglich, einen oder mehrere Zugstäbe analog zu den Formstäben 16 nach Fig. 6 in der nicht dargestellten Ummantelung zu plazieren oder die Elemente 21 als Hohlzylinder auszubilden, in deren Wänden Bohrungen zur Aufnahme eines oder mehrerer Zugfäden ausgebildet sind, die ggf. über Umlenkeinheiten am proximalen Ende verbunden sind. Entscheidend ist in allen Fällen, daß der Druck zwischen den einzelnen Elementen 21 durch den Zug der Zugfäden 26 so groß gewählt werden kann, daß der Formstab 19a insgesamt dauerhaft eine gewünschte Verformung annimmt.

Die anhand der Fig. 9 bis 12 beschriebenen Ausführungsbeispiele bringen den Vorteil mit sich, daß vor dem Zurückziehen des Teils 2d aus dem Tubus durch Verminderung des Anpreßdrucks eine Verminderung der Formstabilltät herbeigeführt werden kann, so daß eine Dislokation des Tubus beim Zurückziehen des Teils 2d unwahrscheinlich wird. Außerdem kann das biegsame Teil 2d in eine Standard-Ausgangsposition gebracht werden, während bei der Anwendung eines Drahts u.U. Restverbiegungen nach einem Biegevorgang zurückbleiben, was bei wiederholten Biegevorgängen zu Beschädigungen der Lichtleiterfasern führen könnte.

Die Erfindung ist nicht auf die beschriebenen Ausführungsbeispiele beschränkt, die auf vielfache Weise abgewandelt werden können. Insbesondere ist es möglich, mehr oder weniger als die dargestellten Faserbündel im formbaren Teil anzuordnen. Weiter werden der Formstab 10 und die Faserbündel 3, 4 und 5 zweckmäßig mit Ummantelungen versehen, damit beim Biege- bzw. Formungsvorgang keine schädlichen Scherkräfte entstehen bzw. auf die empfindlichen Fasern ausgeübt werden. Hierfür eignen sich vor allem solche Materialien, wie sie üblicherweise für die Ummantelung von Endoskopschläuchen verwendet werden. Dabei können zusätzlich äußere Schutzschichten aus Gummi- oder Kunststoff verwendet werden, die ein gutes Gleiten, insbesondere unter Zuhilfenahme eines Gleitmittels ermöglichen und hygienisch einwandfrei sind. Außerdem werden für alle beteiligten Komponenten vorzugsweise solche Materialien verwendet, daß im Teil 2, 2a zumindest bei der bestimmungsgemäßen Anwendung keine Knickstellen entstehen. Weiterhin müssen zumindest die Faserbündel wie bei üblichen Endoskopen so im Teil 2, 2a angeordnet sein, daß sie beim Biegevorgang die erforderlichen axialen Bewegungen relativ zur Ummantelung 9, 15 ausführen können. Weiterhin erstrecken sich die Formstäbe 10, 16 zwar vorzugsweise über die ganze Länge des Teils 2, doch ist es auch möglich, das Teil 2, 2a nur abschnittsweise, insbesondere nur in einem distalen Abschnitt formbar auszubilden. Bei den Formstäben 19, 19a können anstelle der dargestellten Elemente 21 z.B. auch solche verwendet werden, die je zwei konkave Grenzflächen aufweisen, wobei zwischen je zwei solcher Flächen benachbarter Elemente je ein bikonvexes Element oder ein Kugelelement od. dgl. angeordnet ist. Außerdem können die verschiedenen Teile auch in anderen als den beschriebenen und dargestellten Kombinationen verwendet werden. Dabei könnten auch die Zwischenräume insbesondere bei den in Fig. 2 und 8 gezeigten Ausführungsbeispielen mit Füllmaterialen, z.B. Fasern oder Schaumstoff, ausgefüllt sein oder von einem Gas durchströmt werden, das zu den Frontlinsen umgelenkt wird, sofern dies aus hygienischen Gründen möglich ist. Schließlich ist die Erfindung nicht auf die Anwendung des beschriebenen Endoskops für die Intubation beschränkt, sondern kann in entsprechender Abwandlung überall dort eingesetzt werden, wo es erwünscht ist, das Teil 2, 2a in der beschriebenen Weise vorzuformen bzw. an die zu untersuchende Körperhöhlung anzupassen. Dabei versteht sich, daß die Erfindung auch realisiert werden kann, indem der beschriebene Formstab unter Beachtung der obigen Erläuterungen in einem Arbeitskanal eines an sich bekannten Endoskops angeordnet wird. Da in diesem Fall eine Bewegung der Endoskopspitze nicht mehr möglich und auch nicht mehr erwünscht ist, kann auf die hierzu erforderliche Mechanik völlig verzichtet werden.

## Patentansprüche

1. Endoskop mit einem Halteteil (1), mit dem ein flexibles Teil (2, 2a, 2b, 2c) verbunden ist, in dem wenigstens je ein der Beleuchtung dienendes und ein der Betrachtung dienendes, lichtleitendes Faserbündel (3, 3a, 4, 5, 5a, 18) angeordnet sind, wobei das eine mit einem am Halteteil (1) montierten Anschluß (6) für eine Lichtquelle und das andere mit einer am Halteteil (1) montierten Betrachtungsoptik (8) verbunden ist, dadurch gekennzeichnet, daß das Teil (2, 2a, 2b, 2c) zumindest teilweise auch formbar ausgebildet ist.

2. Endoskop nach Anspruch 1, dadurch gekennzeichnet, daß das Teil (2, 2a, 2b, 2c) mit einem Mittel zur Herstellung einer in Abhängigkeit vom Einzelfall vorgewählten Form versehen ist.

3. Endoskop nach Anspruch 2, dadurch gekennzeichnet, daß das Teil (2) eine flexible Umhüllung (9) aufweist, in der die Faserbündel (3, 3a, 4, 5, 5a, 18) und ein Formstab (10, 19, 19a) angeordnet sind.

4. Endoskop nach Anspruch 2 oder 3, dadurch gekennzeichnet, daß das Teil (2a) eine flexible Umhüllung (15) aufweist und in das Umhüllungsmaterial wenigstens ein Formstab (16) eingearbeitet ist.

5. Endoskop nach einem der Ansprüche 2 bis 4, dadurch gekennzeichnet, daß das Mittel aus einer Umhüllung (17) besteht, die aus einem Material mit hoher Formstabilität besteht und die Faserbündel (3, 3a, 5, 5a, 18) aufnimmt.

6. Endoskop nach einem der Ansprüche 3 bis 5, dadurch gekennzeichnet, daß der Formstab (19, 19a) aus einem eine Vielzahl von Elementen (21) aufweisenden Gliederstab besteht, wobei die Elemente (21) aneinandergrenzende, konvexe bzw. konkave Oberflächen (22a, 22b) aufweisen und durch Zug- oder Druckkraft verspannbar sind.
